(19) **Europäisches Patentamt**
**European Patent Office**
**Office européen des brevets**

(11) **EP 2 607 383 B1**

(12) **EUROPEAN PATENT SPECIFICATION**

(45) Date of publication and mention
of the grant of the patent:
**19.12.2018 Bulletin 2018/51**

(21) Application number: **11818075.1**

(22) Date of filing: **03.08.2011**

(51) Int Cl.:
*C08F 2/32* (2006.01)     *C08F 2/20* (2006.01)
*B32B 3/26* (2006.01)     *A61L 15/24* (2006.01)
*A61L 15/60* (2006.01)     *B01J 20/26* (2006.01)
*B32B 5/16* (2006.01)

(86) International application number:
**PCT/JP2011/067748**

(87) International publication number:
**WO 2012/023433 (23.02.2012 Gazette 2012/08)**

(54) **WATER-ABSORBING RESIN**

WASSERABSORBIERENDES HARZ

RÉSINE ABSORBANT L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.08.2010 JP 2010184017**

(43) Date of publication of application:
**26.06.2013 Bulletin 2013/26**

(73) Proprietor: **Sumitomo Seika Chemicals Co., Ltd.
Kako-gun, Hyogo 675-0145 (JP)**

(72) Inventors:
• **TAKATORI, Junichi**
**Himeji-shi**
**Hyogo 672-8076 (JP)**
• **KONDO, Kimihiko**
**Himeji-shi**
**Hyogo 672-8076 (JP)**

• **HANDA, Masayoshi**
**Himeji-shi**
**Hyogo 672-8076 (JP)**

(74) Representative: **Vossius & Partner
Patentanwälte Rechtsanwälte mbB
Siebertstrasse 3
81675 München (DE)**

(56) References cited:
EP-A1- 1 291 368     EP-A1- 2 011 803
EP-A1- 2 048 184     EP-A1- 2 184 300
WO-A1-2004/096304     WO-A1-2006/033477
WO-A1-2008/026783     WO-A1-2008/038840
WO-A1-2010/073658     WO-A1-2010/082373
WO-A2-2004/069915     JP-A- 3 227 301
JP-A- 5 017 509     JP-A- 9 012 613
JP-A- 9 077 810     JP-A- 11 279 207
JP-A- 2006 089 525     JP-A- 2006 131 767
US-A- 5 985 944     US-A1- 2007 015 887

EP 2 607 383 B1

**Description**

Technical Field

The present invention relates to a water-absorbent resin that is suitably used in hygienic materials, and an absorbent material and an absorbent article using the same.

Background Art

In recent years, a water-absorbent resin has been widely used in various fields, including hygienic articles, such as disposable diapers and sanitary napkins, agricultural and horticultural materials, such as water-retaining agents and soil conditioners, and industrial materials, such as water-blocking agents and agents for preventing dew condensation. Of these, a water-absorbent resin is particularly frequently used in hygienic articles, such as disposable diapers and sanitary napkins. As the water-absorbent resin, for example, hydrolysates of starch-acrylonitrile graft copolymers, neutralized products of starch-acrylate graft copolymers, saponified products of vinyl acetate-acrylic ester copolymers, and crosslinked polymers of partially neutralized acrylic acid compound are known.

Absorbent articles such as disposable diapers have a structure in which an absorbent material for absorbing a liquid, e.g., body liquid, is sandwiched between a flexible liquid-permeable surface sheet (top sheet) positioned on a side contacting the body and a liquid-impermeable rear sheet (back sheet) positioned on a side opposite to that contacting the body. The absorbent material is generally made of a mixture of a water-absorbent resin and a hydrophilic fiber.

In recent years, there is increasing demand for thinner or lighter absorbent articles from the viewpoint of design, portability convenience, and transport efficiency, etc. A generally employed method for thinning absorbent articles is, for example, a method in which the amount of hydrophilic fiber, such as refined wood pulp, which serves to secure a water-absorbent resin in an absorbent material, is reduced, and the amount of water-absorbent resin is increased.

An absorbent material that contains a reduced amount of a bulky hydrophilic fiber having a low absorption capacity and an increased amount of a compact water-absorbent resin having a high absorption capacity is intended to reduce the thickness of the absorbent material by reducing the amount of the bulky material while keeping the absorption capacity that corresponds to the design of the absorbent article, and this is a reasonable improvement method. However, when liquid distribution or diffusion that occurs in the actual use of an absorbent material in an absorbent article, e.g., a disposable diaper, is considered, there is a disadvantage in that when a large amount of the water-absorbent resin is formed into a soft gel by liquid absorption, a phenomenon called "gel blocking" occurs, which markedly lowers liquid diffusibility and slows the liquid permeation rate of the absorbent material.

This gel blocking phenomenon is explained below. When an absorbent material containing particularly highly densified water-absorbent resins absorbs liquid, a water-absorbent resin near the surface layer absorbs the liquid to further densify a soft gel around the surface layer, and so liquid permeation into the absorbent material is inhibited, preventing the internal water-absorbent resins from efficiently absorbing the liquid. Accordingly, there has been a demand for a water-absorbent resin that has an appropriate water-absorption rate, is less likely to undergo a gel blocking phenomenon, absorbs liquid, and has high flow-through properties after swelling.

The water-absorbent resin is mainly produced by subjecting a water-soluble ethylenically unsaturated monomer to a reversed-phase suspension polymerization or an aqueous solution polymerization. However, conventional reversed-phase suspension polymerization methods have disadvantages in that water-absorbent resins having a large particle size are less likely to be produced, and an appropriate particle size that is suitable for a hygiene material is less likely to be obtained.

Accordingly, some production methods have been suggested in order to obtain a water-absorbent resin having a large particle size. For example, in a production method according to a reversed-phase suspension polymerization, the following methods have been suggested.

A method comprising producing a water-absorbent resin according to polymerization of a monomer in the first stage, cooling the resin, again adding a monomer to the polymerization reaction solution in which the polymer particles in the first stage are suspended, in a state such that a surfactant is precipitated, and polymerizing the mixture to thereby give a large absorbent resin (Patent Literature 1): a method comprising producing a water-absorbent resin according to polymerization of a monomer in the first stage, again adding a monomer to the polymerization reaction solution in which the polymer particles are suspended, and polymerizing the mixture to thereby agglomerate the polymer particles in the first stage (Patent Literature 2); a method comprising producing a water-absorbent resin according to polymerization of a monomer in the first stage, adding a specific surfactant having an HLB of 7 or more to the polymerization reaction solution in which the polymer particles are suspended, again adding a monomer thereto, and polymerizing the mixture to thereby give a large water-absorbent resin (Patent Literature 3); a method comprising producing a water-absorbent resin according to polymerization of a monomer in the first stage, again adding, in the presence of inorganic powders, a monomer to the polymerization reaction solution in which the polymer particles are suspended, and polymerizing the mixture to thereby give a large water-absorbent resin (Patent Publication 4).

Patent Publication 5 relates to a water-absorbent resin which is obtained by performing a reversed-phase suspension polymerization at two or more stages.

Citation List

Patent Literature

PTL 1: Japanese Unexamined Patent Publication No. H3-227301
PTL 2: Japanese Unexamined Patent Publication (Translation of PCT Application) No. H5-017509
PTL 3: Japanese Unexamined Patent Publication No. H9-012613
PTL 4: Japanese Unexamined Patent Publication (Translation of PCT Application) No. H9-077810
PTL 5: European Patent Application Publication EP 2 184 300 A1

Summary of Invention

Technical Problem

Although water-absorbent resins produced according to conventional technical methods have a large particle size that is suitable for a hygienic material, they have been unable to attain sufficient flow-through properties. The main object of the present invention is to provide a water-absorbent resin that has an appropriate absorption capacity and excellent flow-through properties and that is suitably used in an absorbent article applied to a hygienic material, etc.

Solution to Problem

In view of the object as described above, the present inventors found the following:
When primary particles that have a specific median particle size and are formed by polymerizing at least one water-soluble ethylenically unsaturated monomer according to a reversed-phase suspension polymerization method are agglomerated according to a reversed-phase suspension polymerization method to form a water-absorbent resin having a specific absorption capacity, the water-absorbent resin has excellent flow-through properties and is suitably used in hygienic materials. Specifically, the present invention includes the following embodiments.

A method of producing a water-absorbent resin, comprising the steps of:

(1) polymerizing at least one water-soluble ethylenically unsaturated monomer in the presence of a dispersion stabilizer according to a reversed-phase suspension polymerization method to form a slurry in which primary particles are dispersed, the primary particles having a median particle size of 100 to 250 $\mu$m, and
(2-1) cooling the slurry obtained in Step (1) to precipitate a portion of the dispersion stabilizer contained in the slurry,
(2-2) adding at least one water-soluble ethylenically unsaturated monomer to the slurry obtained in Step (2-1) to perform a polymerization reaction (reversed-phase suspension polymerization method), thereby agglomerating the primary particles dispersed in the slurry, to produce a water-absorbent resin,

wherein the water-absorbent resin has a water-retention capacity of saline solution of 30 g/g or less, wherein the saline solution is a 0.9% by mass sodium chloride aqueous solution, and the water-absorbent resin has a flow-through rate of 0.69% by mass sodium chloride aqueous solution of 200 g/10 minutes or more.

A water-absorbent resin obtainable by this method.

An absorbent material that comprises a hydrophilic fiber and this water-absorbent resin.

An absorbent article including this absorbent material between a liquid-permeable sheet and a liquid-impermeable sheet.

Meanwhile, the present disclosure may be summarized by the following items:

Item 1. A water-absorbent resin formed by agglomerating primary particles according to a reversed-phase suspension polymerization method, the primary particles being obtained by polymerizing at least one water-soluble ethylenically unsaturated monomer in the presence of a dispersion stabilizer according to a reversed-phase suspension polymerization method, the primary particles having a median particle size of 100 to 250 $\mu$m, and the water-absorbent resin having a water-retention capacity of saline solution of 30 g/g or less.

Item 2. The water-absorbent resin according to Item 1, wherein the water-absorbent resin has a flow-through rate of 0.69% by mass sodium chloride aqueous solution of 200 g/10 minutes or more.

Item 3. The water-absorbent resin according to Item 1 or 2, wherein the water-absorbent resin has a water-absorption rate of saline solution of 45 to 180 seconds.

Item 4. The water-absorbent resin according to any one of Items 1 to 3, wherein the dispersion stabilizer is at least one member selected from the group consisting of sucrose fatty acid esters and polyglycerol fatty acid esters.

Item 5. The water-absorbent resin according to any one of Items 1 to 4, wherein the water-soluble ethylenically unsaturated monomer is at least one member selected from the group consisting of (meth)acrylic acid and a salt thereof.

Item 6. An absorbent material that comprises a hydrophilic fiber and the water-absorbent resin according to any one of Items 1 to 5.

Item 7. An absorbent article including the absorbent material according to Item 6 between a liquid-permeable sheet and a liquid-impermeable sheet.

Advantageous Effects of Invention

The present invention can provide a water-absorbent resin having excellent flow-through properties, and the water-absorbent resin of the present invention has an appropriate water-absorption rate. Further, the use of the water-absorbent resin of the present invention can provide an absorbent material having an excellent liquid permeation rate and like properties, and an absorbent article.

Brief Description of Drawings

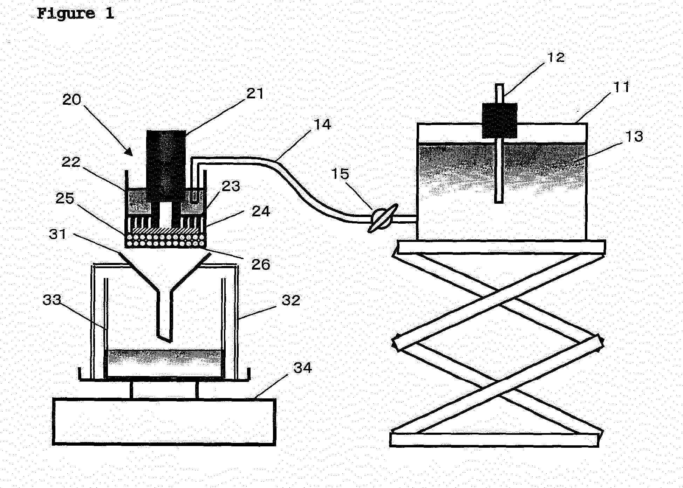

Fig. 1 is a schematic view showing an outline of the constitution of an apparatus for detecting the flow-through rate of 0.69% by mass sodium chloride aqueous solution.

Description of Embodiments

Water-absorbent resin of the present invention

The water-absorbent resin of the present invention can be formed by agglomerating primary particles according to a reversed-phase suspension polymerization method, the primary particles being formed by polymerizing at least one water-soluble ethylenically unsaturated monomer according to a reversed-phase suspension polymerization method.

The primary particles have a median particle size of 100 to 250 $\mu$m, preferably 110 to 240 $\mu$m, and more preferably 130 to 230 $\mu$m. The median particle size can be measured by the method described in the Examples shown below.

To obtain a water-absorbent resin having excellent flow-through properties after liquid absorption and swelling, the primary particles have a median particle size of 100 $\mu$m or more, whereas to prevent reduction in the agglomeration strength and the water-absorption rate of secondary particles, the primary particles have a median particle size of 250 $\mu$m or less.

The shape of the primary particles of the present invention is not particularly limited. However, since the primary particles are formed by a reversed-phase suspension polymerization method, they may be formed into a single particle of substantially spherical shape with a smooth surface, such as a true spherical shape or an elliptic spherical shape. Due to the smooth surface of the primary particles having such a shape, the primary particles have high flowability as powders, and the agglomerated particles are likely to be closely filled. Therefore, even when subjected to a collision, the agglomerated particles are less likely to be broken, and a water-absorbent resin having high particle strength can be obtained.

The primary particles that satisfy the aforementioned range are agglomerated according to a reversed-phase suspension polymerization method to form a water-absorbent resin having a water-retention capacity of saline solution of 30 g/g or less. This resin can be used as the water-absorbent resin of the present invention. To impart more excellent effects to an absorbent article, the water-absorbent resin having a water-retention capacity of saline solution of 25 to 30 g/g is preferably used.

The water-retention capacity of saline solution is defined by the mass of physiological saline that the water-absorbent resin can absorb (per unit mass) and indicates the degree of the liquid absorption capacity of the water-absorbent resin. The water-retention capacity of saline solution can be measured by the method described in the Examples shown below.

The water-absorbent resin of the present invention that satisfies the numerical ranges of the median particle size and the water-retention capacity of saline solution described above can prevent a phenomenon in which liquid diffusion is inhibited, i.e., gel blocking, when used as an absorbent material. Further, the water-absorbent resin of the present invention can prevent returning of the absorbed liquid and liquid leakage when used in an absorbent material.

One of the indices that feature the water-absorbent resin of the present invention is the flow-through rate of 0.69% by mass sodium chloride aqueous solution. Specifically, the water-absorbent resin generally has a flow-through rate of 0.69% by mass sodium chloride aqueous solution of 200 g or more, preferably 200 to 800 g, and more preferably 250 to 700 g per 10 minutes.

The flow-through rate of 0.69% by mass sodium chloride aqueous solution is defined by the amount of 0.69% by mass sodium chloride aqueous solution (per unit time) that passes through a water-absorbent resin (per unit mass) that has been swelled by liquid absorption, and indicates the degree of the flow-through properties of the water-absorbent resin after liquid absorption. The flow-through rate of 0.69% by mass sodium chloride aqueous solution can be measured by the method described in the Examples shown below.

One of the indices that feature the water-absorbent resin of the present invention is the water-absorption rate of saline solution. Specifically, the water-absorption rate of saline solution is generally 45 to 180, preferably 50 to 170, and more preferably 60 to 150 seconds.

The water-absorption rate of saline solution indicates a time until the water-absorbent resin absorbs a predetermined amount of physiological saline (per unit mass). The water-absorption rate of saline solution can be measured by the method described in the Examples shown below.

The water-absorbent resin has a median particle size of 250 $\mu$m or more (preferably 300 $\mu$m or more) to prevent a phenomenon in which liquid diffusion is inhibited, i.e., gel blocking, when used in an absorbent material, etc. The water-absorbent resin has a median particle size of 600 $\mu$m or less (preferably 500 $\mu$m or less) to prevent the resin from becoming partially rigid and to maintain flexibility when used in an absorbent material. The median particle size can be measured by the method described in the Examples shown below.

The water-absorbent resin of the present invention generally has a water content of 20% by mass or less, and preferably 5 to 10% by mass to prevent reduction in flowability as powders. The water content can be measured by the method described in the Examples shown below.

Method for producing the water-absorbent resin of the present invention

The method of the present invention is performed by a reversed-phase suspension polymerization reaction of at least two stages. Specifically, the method includes the following Steps 1 and 2.

(1) Step 1, in which at least one water-soluble ethylenically unsaturated monomer is polymerized according to a reversed-phase suspension polymerization method in the presence of a dispersion stabilizer to form a slurry in which primary particles are dispersed; and
(2) Step 2, in which at least one water-soluble ethylenically unsaturated monomer is added to the slurry obtained in Step 1 to perform a polymerization reaction (reversed-phase suspension polymerization method), thereby agglomerating the primary particles dispersed in the slurry.

Since the agglomeration obtained in Step 2 is also obtained in the state of a slurry, to enhance the production efficiency, a step using a reversed-phase suspension polymerization method, in which a polymerization reaction is performed by further adding a water-soluble ethylenically unsaturated monomer to the slurry in which the agglomeration obtained in Step 2 is dispersed, can be repeated to form an agglomeration, thereby obtaining a water-absorbent resin.

An embodiment of a method of producing the water-absorbent resin of the present invention is detailed below; however, the present invention is not limited to this embodiment.

Step 1

The reversed-phase suspension polymerization method used in this step is a method in which a water-soluble ethylenically unsaturated monomer-containing aqueous solution is added to a dispersion stabilizer-containing dispersion medium to conduct suspension by stirring, thereby polymerizing the water-soluble ethylenically unsaturated monomer in the dispersed aqueous solution. By this method, the slurry including the dispersed primary particles that are produced by polymerizing the monomer in the dispersion medium and that satisfy the range of the mediate particle size described above can be obtained in Step 1.

Examples of the water-soluble ethylenically unsaturated monomer used include (meth)acrylic acid (herein "acryl" and "methacryl" collectively refer to "(meth)acryl," and "acrylate" and "methacrylate" collectively refer to "(meth)acrylate"), 2-(meth)acrylamide-2-methylpropanesulfonic acid, and salts thereof; nonionic monomers, such as (meth)acrylamide, N,N-dimethyl(meth)acrylamide, 2-hydroxyethyl(meth)acrylate, N-methylol(meth)acrylamide, and polyethylene glycol mono(meth)acrylate; amino-group-containing unsaturated monomers, such as N,N-diethylaminoethyl (meth)acrylate, N,N-diethylaminopropyl(meth)acrylate, diethylaminopropyl(meth)acrylamide, and quaternary compounds thereof.

At least one member selected from these groups can be used. Of these, acrylic acid, methacrylic acid, acrylate, methacrylate, acrylamide, methacrylamide, and N,N-dimethylacrylamide are preferably used.

The concentration of water-soluble ethylenically unsaturated monomer in the monomer-containing aqueous solution is not particularly limited. It is preferable that the concentration of the monomer in the monomer-containing aqueous solution be 20% by mass to a saturated concentration, more preferably 25 to 70% by mass, and even more preferably 30 to 55% by mass.

When a monomer containing an acid group such as (meth)acrylic acid or 2-(meth)acrylamide-2-methylpropanesulfonic acid is used as the water-soluble ethylenically unsaturated monomer, the acid group may be neutralized in advance with an alkaline neutralizing agent, such as an alkali metal salt. Examples of the alkaline neutralizing agent include aqueous solutions of sodium hydroxide, potassium hydroxide, and ammonia. These alkaline neutralizing agents can be used alone or in a combination of two or more.

The degree of neutralization of all the acid groups with the alkaline neutralizing agent is generally in the range of 10 to 100% by mol, and preferably in the range of 30 to 80% by mol, from the viewpoint of increasing osmotic pressure of the resulting water-absorbent resin to increase absorption properties, and not causing any disadvantages in safety or the like due to the presence of an excess alkaline neutralizing agent.

As a dispersion medium, a petroleum hydrocarbon dispersion medium can be used. Examples of the petroleum hydrocarbon solvent include aliphatic hydrocarbons, such as n-hexane, n-heptane, n-octane, and ligroin; alicyclic hydrocarbons, such as cyclopentane, methylcyclopentane, cyclohexane, and methylcyclohexane; and aromatic hydrocarbons, such as benzene, toluene, and xylene. These dispersion media can be used alone or in a combination of two or more. Of these, n-hexane, n-heptane, and cyclohexane are preferably used because they are easily available industrially, stable in quality, and inexpensive. From the viewpoint of the same, as an example of a combination of the above, Exxsol™ Heptane Fluid are commercially available (produced by ExxonMobil Co., Ltd.: containing 75-85% by mass n-heptane and isomers) are preferably used .

As a dispersion stabilizer, a surfactant can be used. Examples of the surfactant include sucrose fatty acid esters, polyglycerol fatty acid esters, sorbitan fatty acid esters, polyoxyethylene sorbitan fatty acid esters, polyoxyethylene glycerol fatty acid esters, sorbitol fatty acid esters, polyoxyethylene sorbitol fatty acid esters, polyoxyethylene alkyl ethers, polyoxyethylene alkylphenyl ethers, polyoxyethylene castor oil, polyoxyethylene cured castor oil, alkylallylformaldehyde condensed polyoxyethylene ethers, polyoxyethylene polyoxypropylene block copolymers, polyoxyethylene polyoxypropyl alkyl ethers, polyethylene glycol fatty acid esters, alkyl glucosides, N-alkyl glucone amides, polyoxyethylene fatty acid amides, polyoxyethylene alkylamines, phosphoric esters of polyoxyethylene alkyl ethers, and phosphoric esters of polyoxyethylene alkylallyl ethers. Of these, from the viewpoint of dispersion stability of an aqueous monomer solution, sucrose fatty acid esters and polyglyceryl fatty acid esters are preferably used. These dispersion stabilizers can be used alone or in a combination of two or more.

The HLB of a surfactant used as a dispersion stabilizer affects the shape of the resulting primary particles. The HLB of a surfactant that is selected to obtain a water-absorbent resin having a substantially spherical primary particle shape as mentioned above cannot be generalized because it varies depending on the kind of surfactant used. For example, in the case of a sucrose fatty acid ester, a surfactant having an HLB of 5 or less can be used.

Together with a surfactant, a polymeric dispersion agent can be used as a dispersion stabilizer. Examples of the polymeric dispersion agent used include maleic anhydride-modified polyethylene, maleic anhydride-modified polypropylene, maleic anhydride-modified ethylene-propylene copolymers, maleic anhydride-modified EPDM (ethylene-propylene-diene terpolymer), maleic anhydride-modified polybutadiene, ethylene-maleic anhydride copolymers, ethylene-propylene-maleic anhydride copolymers, butadiene-maleic anhydride copolymers, oxidized polyethylene, ethylene-acrylic acid copolymers, ethyl cellulose, and ethyl hydroxyethyl cellulose. Of these, maleic anhydride-modified polyethylene, maleic anhydride-modified polypropylene, maleic anhydride-modified ethylene-propylene copolymers, oxidized polyethylene, and ethylene-acrylic acid copolymers are preferably used from the viewpoint of dispersion stability of an aqueous monomer solution. These polymeric dispersions agents can be used alone or in a combination of two or more.

The dispersion stabilizer is generally used in an amount of 0.1 to 5 parts by mass, and preferably 0.2 to 3 parts by mass based on 100 parts by mass of the monomer-containing aqueous solution to keep an excellent dispersion state of the monomer-containing aqueous solution in the petroleum hydrocarbon dispersion medium, which is used as a dispersion medium, and to obtain a dispersion effect that corresponds to the amount used.

A crosslinking agent (internal crosslinking agent) may be added, if necessary, to the monomer-containing aqueous solution to carry out polymerization. As the internal crosslinking agent that is added to the aqueous monomer solution prior to monomer polymerization reaction, compounds having two or more polymerizable unsaturated groups can be used. Examples of the crosslinking agent include di- or tri-(meth)acrylate esters of polyols such as (poly)ethylene glycol (for example, "polyethylene glycol" and "ethylene glycol" as used herein are collectively refer to "(poly)ethylene glycol"), (poly)propylene glycol, trimethylolpropane, glycerol polyoxyethylene glycol, polyoxypropylene glycol, and (poly)glycerol; unsaturated polyesters obtained by reacting the above-mentioned polyol with an unsaturated acid, such as maleic acid and fumaric acid; bisacrylamides, such as N,N'-methylenebis(meth)acrylamide; di- or tri(meth)acrylate esters obtained by reacting a polyepoxide with (meth)acrylic acid; carbamyl esters of di(meth)acrylic acid obtained by reacting a polyisocyanate, such as tolylenediisocyanate or hexamethylenediisocyanate, with hydroxyethyl(meth)acrylate; allylated starch; allylated cellulose; diallyl phthalate; N,N',N''-triallyl isocyanurate; and divinylbenzene.

As the internal crosslinking agent, in addition to the aforementioned compounds having two or more polymerizable unsaturated groups, compounds having two or more other reactive functional groups can be used. Examples thereof include glycidyl-group-containing compounds, such as (poly)ethylene glycol diglycidyl ethers, (poly)propylene glycol diglycidyl ethers, and (poly)glycerol diglycidyl ethers; (poly)ethylene glycol; (poly)propylene glycol; (poly)glycerol; pentaerythritol; ethylenediamine; polyethyleneimine; and glycidyl(meth)acrylate.

These internal crosslinking agents can be used alone or in a combination of two or more.

The amount of the internal crosslinking agent added is generally 1% by mol or less, and preferably 0.5% by mol or less, based on the total amount of the monomer used in this step from the viewpoint of sufficiently enhancing the absorption capacity of the resulting water-absorbent resin.

To control absorption properties of the water-absorbent resin, a chain transfer agent may be added to the monomer-containing aqueous solution. Examples of the chain transfer agent include hypophosphites, thiols, thiolic acids, secondary alcohols, amines, and the like.

The monomer-containing aqueous solution may contain a radical polymerization initiator. Examples of the radical polymerization initiator include persulfates, such as potassium persulfate, ammonium persulfate, and sodium persulfate; peroxides such as, methyl ethyl ketone peroxide, methyl isobutyl ketone peroxide, di-t-butyl peroxide, t-butyl cumyl peroxide, t-butyl peroxyacetate, t-butyl peroxyisobutylate, t-butyl peroxypivalate, and hydrogen peroxide; and azo compounds, such as 2,2'-azobis(2-amidinopropane)dihydrochloride, 2,2'-azobis[2-(N-phenylamidino)propane]dihydrochloride, 2,2'-azobis[2-(N-allylamidino)propane]dihydrochloride, 2,2'-azobis{2-[1-(2-hydroxyethyl)-2-imidazoline-2-yl]propane}dihydrochloride, 2,2'-azobis {2-methyl-N-[1,1-bis(hydroxymethyl)-2-hydroxyethyl]propionamide}, 2,2'-azobis[2-methyl-N-(2-hydroxyethyl)-propionamide], and 4,4'-azobis(4-cyanovaleric acid). These radical polymerization initiators can be used alone or in a combination of two or more.

The radical polymerization initiator is generally used in an amount of 0.005 to 1% by mol, based on the total amount of the monomer used in this step. The radical polymerization initiator in an amount less than 0.005% by mol is not preferable because monomer polymerization reaction requires a long period of time, and the radical polymerization initiator in an amount more than 1% by mol is not preferable because an abrupt polymerization reaction occurs.

The radical polymerization initiator can be also used as a redox polymerization initiator together with a reducing agent, such as sodium sulfite, sodium hydrogen sulfite, ferrous sulfate, and L-ascorbic acid.

The reaction temperature of the monomer polymerization reaction varies depending on the presence or absence of a radical polymerization initiator and the kind of a radical polymerization initiator used. The reaction temperature is generally 20 to 110°C, and preferably 40 to 90°C. By performing the monomer polymerization reaction at a temperature in this range, the monomer polymerization reaction can be terminated within an appropriate time. In addition, since heat generated in the monomer polymerization reaction can be easily removed, the polymerization reaction proceeds smoothly. The reaction time is generally 0.1 to 4 hours.

The median particle size of the primary particles produced in Step 1 can be adjusted, for example, by changing the rotational speed of stirring during monomer polymerization reaction using various kinds of stirring impellers. As the stirring impellers, a propeller impeller, a paddle impeller, an anchor impeller, a turbine impeller, a Phaudler impeller, a ribbon impeller, a Fullzone impeller (produced by Shinko Pantec Co., Ltd.), a MAXBLEND impeller (produced by Sumitomo Heavy Industries, Ltd.), and a Super-Mix impeller (produced by Satake Chemical Equipment Mfg., Ltd.) can be used. In general, when the same kinds of stirring impellers are used, the higher the rotational speed of stirring, the smaller the particle size of the resulting primary particles.

The median particle size of the primary particles can be also adjusted by adding a thickener to the water-soluble ethylenically unsaturated monomer-containing aqueous solution to change the viscosity of the aqueous solution. Examples of the thickener include hydroxyethyl cellulose, hydroxypropyl cellulose, methyl cellulose, carboxymethyl cellulose, polyacrylic acid, (partially) neutralized products of polyacrylic acid, polyethylene glycol, polyacrylamide, polyethylene imine, dextrin, sodium alginate, polyvinyl alcohol, polyvinyl pyrrolidone, and polyethylene oxide. In general, when the rotational speeds of stirring are the same, the higher the viscosity of the aqueous monomer solution, the larger the particle size of the resulting primary particles.

As mentioned above, the median particle size of the primary particles of the present invention can be controlled by adjusting the rotational speed of stirring or the viscosity of a water-soluble ethylenically unsaturated monomer-containing aqueous solution.

Step 2

In step 2, an aqueous solution containing a water-soluble ethylenically unsaturated monomer is added to the slurry obtained in step 1 in which the primary particles are dispersed so as to cause a polymerization reaction of the monomer, i.e., a reversed-phase suspension polymerization method, thereby agglomerating the primary particles dispersed in the slurry.

Step 2 may comprise a step of cooling the slurry obtained in step 1 to precipitate a portion of the dispersion stabilizer contained in the slurry, before carrying out the polymerization reaction of the monomer. The cooling temperature is not particularly limited, and is usually 10 to 50°C. Precipitation of the dispersion stabilizer can be confirmed by the presence of white turbidity in the slurry. Means such as visual observation and a turbidity meter can be used to confirm the turbidity.

Specifically, the addition of an aqueous solution containing the water-soluble ethylenically unsaturated monomer in step 2 is carried out in order to agglomerate the primary particles obtained by the polymerization in step 1 to obtain a particle size suitable for use in hygienic materials.

The same monomers mentioned as examples of the water-soluble ethylenically unsaturated monomer used in step 1 can be used as the water-soluble ethylenically unsaturated monomer in step 2. The type of monomers, degree of neutralization, type of salts formed by neutralization, and concentration of the monomer in the aqueous solution may be the same or different from those of the water-soluble ethylenically unsaturated monomer used in step 1.

A polymerization initiator may be added to the aqueous solution containing the water-soluble ethylenically unsaturated monomer used in step 2. The polymerization initiator to be used can be suitably selected from the examples of the polymerization initiator used for the polymerization in step 1.

The water-soluble ethylenically unsaturated monomer used in step 2 is usually added in an amount of 50 to 300 parts by mass, preferably 100 to 200 parts by mass, more preferably 120 to 160 parts by mass, relative to 100 parts by mass of the water-soluble ethylenically unsaturated monomer used in step 1, from the viewpoint of obtaining a water-absorbent resin that forms an appropriate agglomeration.

Further, an internal crosslinking agent and a chain transfer agent may be added, if necessary, to the aqueous solution containing the water-soluble ethylenically unsaturated monomer used in step 2. Usable agents can be selected from the examples mentioned in step 1. These agents can be used in the same amount defined in step 1.

Although the polymerization reaction temperature of the monomer in step 2 varies depending on the radical polymerization initiator to be used, the temperature is usually 20 to 110° C, preferably 40 to 90° C. The reaction time is usually from 0.1 hours to 4 hours.

Stirring in the reversed-phase suspension polymerization method in step 2 is carried out to homogeneously mix the entire solution.

The median particle size of the water-absorbent resin obtained by the above method can be suitably adjusted by controlling the precipitation state of the dispersion stabilizer and the amount of water-soluble ethylenically unsaturated monomer in step 2 relative to the amount of water-soluble ethylenically unsaturated monomer used in step 1.

Step 2 may comprise a step of treating the primary particles by adding a crosslinking agent (post-crosslinking agent) thereto after the primary particles are agglomerated. This step results in an increase in the surface crosslinking density of the resulting agglomeration, and allows the production of a water-absorbent resin excellent in various properties such as water absorption capacity under load, water-absorption rate, gel strength, and liquid absorption capacity; and which is suitable for use in hygienic materials.

Compounds having two or more reactive functional groups are exemplified as such a post-crosslinking agent. Examples thereof include diglycidyl group-containing compounds such as (poly)ethylene glycol diglycidyl ether, (poly)glycerol (poly)glycidyl ether, (poly)propylene glycol diglycidyl ether, and (poly)glycerol diglycidyl ether; (poly)ethylene glycol; (poly)propylene glycol; (poly)glycerol; pentaerythritol; ethylenediamine; polyethyleneimine. Of these post-crosslinking agents, (poly)ethylene glycol diglycidyl ether, (poly)propylene glycol diglycidyl ether, and (poly)glycerol diglycidyl ether are suitably used. These post-crosslinking agents may be used singly, or in a combination of two or more.

The post-crosslinking agent is added in an amount that is usually in the range of from 0.005 to 1 % by mol, preferably in the range of from 0.01 to 0.5 % by mol, relative to the total amount of the monomer used, from the viewpoint of not lowering the absorption capacity of the resulting water-absorbent resin, and increasing a crosslinking density on its surface or near its surface to enhance the various properties described above.

The timing for adding the post-crosslinking agent is not particularly limited, and the post-crosslinking agent is added in the presence of usually 1 to 400 parts by mass of water, preferably 5 to 200 parts by mass of water, more preferably 10 to 100 parts by mass of water, relative to 100 parts by mass of the total amount of the water-soluble ethylenically unsaturated monomer used.

The timing for adding the post-crosslinking agent is selected in accordance with the amount of water in the water-absorbent resin, whereby the water-absorbent resin can be more suitably subjected to crosslinking on its surface or near its surface, and the resulting agglomeration as the water-absorbent resin can be provided with a suitable absorption property.

Further, although it depends on the median particle size of the primary particles, it is possible to control the liquid absorption capacity of the water-absorbent resin in accordance with the above-described timing for adding the post-crosslinking agent. For example, it is observed that if the post-crosslinking treatment is carried out when the water-absorbent resin has low water content, the resulting water-absorbent resin tends to have a greater absorption capacity.

When the post-crosslinking agent is added, the post-crosslinking agent may be added as it is, or added in a form of an aqueous solution. If necessary, the hydrophilic organic solvent may be used as a solvent. Examples of hydrophilic organic solvents include lower alcohols such as methyl alcohol, ethyl alcohol, n-propyl alcohol and isopropyl alcohol; ketones such as acetone and methyl ethyl ketone; ethers such as diethyl ether, dioxane, and tetrahydrofuran; amides such as N,N-dimethylformamide; and sulfoxides such as dimethyl sulfoxide.

These hydrophilic organic solvents may be used singly, or in a combination of two or more. Alternatively, these hydrophilic organic solvents may be used as a mixed solvent with water.

The reaction temperature in the post-crosslinking treatment is preferably 50 to 250° C, more preferably 60 to 180° C, further preferably 60 to 140° C, and still further preferably 70 to 120° C. The reaction time is usually from 0.1 hours to 5 hours.

After the post-crosslinking treatment is carried out as described above, drying is performed, and the water-absorbent resin is thereby obtained.

The drying may be carried out under normal pressure or reduced pressure, and may also be carried out under a nitrogen stream in order to increase the drying efficiency. When the drying is carried out under normal pressure, the drying temperature is preferably 70 to 250°C, more preferably 80 to 180° C, further preferably 80 to 140°C, and still further preferably 90 to 130° C. Further, when the drying is carried out under reduced pressure, the drying temperature is preferably 60 to 100°C, more preferably 70 to 90°C.

The thus-obtained water-absorbent resin of the present invention has an excellent flow-through property, and exerts an action of inhibiting gel blocking phenomenon when used as an absorbent material. The absorbent material is suitably used in an absorbent article.

Absorbent Material and Absorbent Article of the Present Invention

The absorbent material of the present invention comprises the above-described water-absorbent resin and hydrophilic fibers. Examples of the structure of the absorbent material include a mixed dispersant obtained by mixing water-absorbent resins and hydrophilic fibers to form a homogeneous composition; a sandwich structure in which the water-absorbent resins are sandwiched between layered hydrophilic fibers; and a structure in which the water-absorbent resins and hydrophilic fibers are wrapped with tissue paper. However, the present invention is not limited to these examples.

Adhesive binders such as thermal-fusible synthetic fibers, hot melt adhesive, and adhesive emulsion may be added to the absorbent material in order to improve the shape-retaining property of the absorbent material.

Examples of hydrophilic fibers include cellulose fibers such as cotton-like pulp obtained from wood, mechanical pulp, chemical pulp, and semi-chemical pulp; artificial cellulose fibers such as rayon and acetate; and fibers formed from synthetic resins such as hydrophilic-treated polyamide, polyester, and polyolefin.

The absorbent article of the present invention has a structure in which the above-described absorbent material is included between a liquid-permeable sheet and a liquid-impermeable sheet.

Examples of liquid-permeable sheets include nonwoven fabrics such as air-through-type, spun bond-type, chemical bond-type and needle punch-type, which are made of fibers such as polyethylene, polypropylene, and polyester.

Examples of liquid-impermeable sheets include synthetic resin films made of a resin such as polyethylene, polypropylene, and polyvinyl chloride.

The type of absorbent articles is not particularly limited. Representative examples of absorbent articles include hygienic materials such as disposal diapers, sanitary napkins, and incontinence pads; urine-absorbing materials for pets; materials for civil engineering and construction such as packing materials; food freshness preservers such as drip absorbents and refrigerants; and agricultural and horticultural materials such as water-retaining materials for soil.

The water-absorbent resin of the present invention shows effects such as a high liquid permeation time and a small re-wet when used as an absorbent material because the median particle size of the primary particles is suitable and the flow-through rate, which indicates the diffusivity of liquid after the resin has absorbed the liquid and swollen, is excellent, as described above.

EXAMPLES

The present invention is described in further detail below based on Examples and Comparative Examples. However, the present invention is not limited to these Examples.

The water-absorbent resin obtained in each Example and Comparative Example was evaluated in terms of the median particle size, water content, water-retention capacity of saline solution, water-absorption rate of saline solution, and flow-through rate of 0.69% by mass sodium chloride aqueous solution by the following methods.

<Median Particle Size>

The particle size of the water-absorbent resin was defined as the median particle size and measured as follows, unless otherwise specified. 0.25 g of amorphous silica (Degussa Japan, Sipernat 200) was mixed as a lubricant with 50 g of a water-absorbent resin.

The above-mentioned water-absorbent resin were allowed to pass through a JIS standard sieve having an opening of 250 μm. When 50% by mass or more of the water-absorbent resin passed through the JIS standard sieve, the median particle size was measured using a combination of sieves of (A). When less than 50% by mass of the water-absorbent resin passed through the JIS standard sieve, the median particle size was measured using a combination of sieves of (B).

(A) JIS standard sieves having openings of 425 μm, 250 μm, 180 μm, 150 μm, 106 μm, 75 μm, and 45 μm, and a receiving tray were combined in that order from the top.
(B) JIS standard sieves having openings of 850 μm, 600 μm, 500 μm, 425 μm, 300 μm, 250 μm, and 150 μm, and a receiving tray were combined in that order from the top.

The above-mentioned water-absorbent resin were placed on the top sieve of the combined sieves, and shaken for 20 minutes using a Ro-Tap Sieve Shaker to sort the water-absorbent resin.

After sorting, the mass of the water-absorbent resin remaining on each sieve was calculated as the mass percentage relative to the total mass, and the mass percentage was integrated in descending order of particle size. Thereby, the relationship between the sieve opening and the integrated value of the mass percentage of the water-absorbent resin remaining on the sieve was plotted on a logarithmic probability paper. By connecting the plots on the probability paper with a straight line, the particle size corresponding to the 50% percentile of the integrated mass percentage was obtained as the median particle size.

<Water Content>

About 2 g of the water-absorbent resin was precisely weighed out (Wa (g)) in a previously weighed aluminum foil case (No. 8). The above sample was dried for 2 hours in a hot air oven (produced by ADVANTEC) set at an internal temperature of 105°C. Thereafter, the dried sample was allowed to cool in a desiccator, and the mass Wb (g) of the dried water-absorbent resin was determined. The water content of the water-absorbent resin was calculated by the following formula:

$$\text{Water content (\%)} = [Wa-Wb]/Wa \times 100$$

<Water-retention capacity of saline solution >

500 g of 0.9% by mass sodium chloride aqueous solution (physiological saline) was weighed out in a 500-mL beaker. 2.0 g of water-absorbent resin was dispersed therein while stirring the solution at 600 r/min so as not to form lumps. The solution was left to stand for 30 minutes in a stirred state to sufficiently swell the water-absorbent resin. Subsequently, the solution was poured into a cotton bag (Cottonbroad No. 60, 100 mm (W) x 200 mm (H)), and the upper part of the cotton bag was tied with a rubber band. The cotton bag was dehydrated for 1 minute using a dehydrator (produced by Kokusan Co., Ltd., product number: H-122) set to produce a centrifugal force of 167 G, and the mass Wc (g) of the dehydrated cotton bag containing swollen gel was measured. The same procedure was repeated without adding the water-absorbent resin, and the mass Wb (g) of the empty cotton bag when wet was measured. The retention capacity was calculated by the following formula:

$$\text{Water-retention capacity of saline solution (g/g)} = [Wc-Wd](g)/\text{mass of the water-absorbent resin (g)}$$

<Water-Absorption Rate of Saline Solution >

This test was carried out in a room whose temperature was controlled to 25°C ± 1°C. 50±0.1 g of physiological saline was weighed out in a 100-mL beaker, and a magnetic stirrer bar (8 mmφ × 30 mm, without a ring) was placed therein. The beaker was immersed in a constant temperature water bath, and the liquid temperature was adjusted to

$25\pm0.2°C$. Next, the beaker was placed on the magnetic stirrer bar, and a vortex was formed in the physiological saline at a rotation speed of 600 r/min. Subsequently, $2.0 \pm 0.002$ g of the water-absorbent resin was then quickly added to the beaker; and, using a stopwatch, the time period (seconds) from a point of addition of the water-absorbent resin to a point of convergence of a vortex on the liquid surface was measured as the absorption rate of the water-absorbent resin.

<Flow-Through Rate of 0.69% by Mass Sodium Chloride Aqueous Solution >

(a) Preparation of Synthetic Urine

Potassium chloride (2 g), anhydrous sodium sulfate (2 g), calcium chloride (0.19 g), magnesium chloride (0.23 g), ammonium dihydrogen phosphate (0.85 g), ammonium monohydrogen phosphate(0.15 g), and distilled water (q.s.) were placed in a 1-L container, and completely dissolved in the distilled water. Distilled water was further added to adjust the total volume of the aqueous solution to 1 L.

(b) Installation of measuring device

The measuring device used shows in Figure 1. In the device, a glass tube for static pressure adjustment 12 was inserted into the tank 11, and a lower end of the glass tube 12 was disposed so that 0.69% by mass sodium chloride aqueous solution 13 was positioned 5 cm higher than a bottom of the swelling gel 25 in the cylinder 22. 0.69% by mass sodium chloride aqueous solution 13 contained in the tank 11 was supplied to the cylinder 22 via an L-shaped tube having cock 14. A collecting container 33 for collecting liquid having passed through the gel layer was disposed under the cylinder 22, and the collecting container 33 was placed on an even balance 34. An inside diameter of the cylinder 22 was 6 cm, and No.400 stainless metal gauze ($38\mu m$ in mesh) 26 was placed on a bottom of a lower portion of the cylinder 22. A hole 23 which allowed liquid to pass through was provided on a lower portion of a piston-type weight 21, and a glass filter 24 having high permeability was provided on the bottom thereof so that the water-absorbent resin or the swelling gel did not enter into the hole 23.

(c) Measurement of Flow-Through Rate

0.9 g of water-absorbent resin was homogeneously placed in a cylindrical container 20, and the water-absorbent resin was swelled in synthetic urine for 60 minutes under increased pressure of 2.07 KPa, thereby forming a gel layer 25. Next, under increased pressure of 2.07 KPa, a 0.69% by mass sodium chloride aqueous solution 13 was flowed through the swollen gel layer 25 from a tank 11 at constant hydrostatic pressure which could position 0.69% by mass sodium chloride aqueous solution 13 to 5 cm higher than a bottom of the swelling gel 25 in the cylinder 22. The mass of the 0.69% by mass sodium chloride aqueous solution 13 that flowed into a collecting container 33 through the gel layer 25 within 10 minutes from the start of flowing was measured as the flow-through rate([g/10 minutes]). The measurement was carried out at room temperature (20 to 25° C).

Example 1

A 2-L cylindrical round-bottomed separable flask having an internal diameter of 110 mm, equipped with a reflux condenser, a dropping funnel, a nitrogen gas inlet tube, and a stirring impeller having 2 sets of four pitched paddle impellers (a impeller diameter of 50 mm) as a stirrer was provided. This flask was charged with 340 g of n-heptane, and 0.92 g of a sucrose stearate having an HLB of 3 (produced by Mitsubishi-Kagaku Foods Corporation, Ryoto sugar ester S-370) and 0.92 g of a maleic anhydride-modified ethylene-propylene copolymer (produced by Mitsui Chemicals, Inc., Hi-wax 1105A) were added thereto. The temperature was raised to 80°C under stirring to dissolve the surfactant, and thereafter the solution was cooled to 50° C.

Separately, a 500-mL Erlenmeyer flask was charged with 92 g (1.02 mol) of 80% by mass acrylic acid aqueous solution, and 146.0 g of 21% by mass of sodium hydroxide aqueous solution was added dropwise thereto under external cooling to neutralize 75 % by mol. Thereafter, 0.11 g (0.41 mmol) of potassium persulfate as a radical polymerization initiator and 9.2 mg (0.06 mmol) of N,N'-methylenebisacrylamide as an internal crosslinking agent were added thereto and dissolved, thereby preparing an aqueous monomer solution for the first step.

Setting the rotation speed of the stirrer at 350 r/min, the entire amount of aqueous monomer solution for the first step was added to the separable flask, and the inside of the system was sufficiently replaced with nitrogen. Thereafter, the flask was immersed in a water bath at 70°C to raise the temperature, and the first-step polymerization was carried out for 30 minutes, followed by cooling to room temperature, thereby obtaining polymerized liquid slurry for the first step. (Note that this polymerized slurry was subjected to azeotropic distillation of water and n-heptane using an oil bath at 120°C to distill off only water to the outside of the system. Subsequently, n-heptane was evaporated, and the slurry was dried. The resulting spherical primary particles had a median particle size of 110 pm.)

Separately, another 500-mL Erlenmeyer flask was charged with 128.8 g (1.43 mol) of 80% by mass acrylic acid aqueous solution, and 159.0 g of 27% by mass sodium hydroxide aqueous solution was added dropwise thereto under external cooling to neutralize 75 % by mol. Thereafter, 0.16 g (0.59 mmol) of potassium persulfate as a radical polymerization initiator and 12.9 mg (0.08 mmol) of N,N'-methylenebisacrylamide as an internal crosslinking agent were added thereto and dissolved, thereby preparing an aqueous monomer solution for the second step.

After the rotation speed of the stirrer was changed to 1,000 r/min and the inside of the system was cooled to 24°C, the entire amount of aqueous monomer solution for the second step was added to the polymerized liquid slurry for the first step, and the inside of the system was sufficiently replaced with nitrogen. Thereafter, the flask was again immersed in a water bath at 70° C to raise the temperature, and the second-step polymerization was carried out for 30 minutes.

After the second-step polymerization, the temperature was raised using an oil bath at 125°C, and the slurry was subjected to azeotropic distillation of water and n-heptane to distill off 222 g of water to the outside of the system while refluxing n-heptane. Thereafter, 3.97 g (0.64 mmol) of a 2% by mass aqueous solution of ethylene glycol diglycidyl ether was added thereto, and the mixture was maintained at 80° C for 2 hours. Subsequently, n-heptane was evaporated, and the slurry was dried, thereby obtaining 230.2 g of water-absorbent resin (A) in the form of agglomeration of spherical particles. The water-absorbent resin had a median particle size of 386 $\mu$m and a water content of 8.2%. Table 1 shows the measurement results of each property.

Example 2

The same procedure as in Example 1 was repeated except that the rotation speed of the stirrer for the first step was changed to 300 r/min, thereby obtaining 233.5 g of water-absorbent resin (B) in the form of agglomeration of spherical particles. The primary particles had a median particle size of 130 $\mu$m, and the water-absorbent resin had a median particle size of 415 $\mu$m and a water content of 7.3%. Table 1 shows the measurement results of each property.

Example 3

The same procedure as in Example 2 was repeated except that the amount of dehydration by refluxing n-heptane after the second-step polymerization was changed to 217 g, thereby obtaining 232.9 g of water-absorbent resin (C) in the form of agglomeration of spherical particles. The primary particles had a median particle size of 130 $\mu$m, and the water-absorbent resin had a median particle size of 424 $\mu$m and a water content of 10.0%. Table 1 shows the measurement results of each property.

Example 4

A 2-L cylindrical round-bottomed separable flask having an internal diameter of 110 mm, equipped with a reflux condenser, a dropping funnel, a nitrogen gas inlet tube, and a stirring impeller having 2 sets of four pitched paddle impellers (a impeller diameter of 50 mm) as a stirrer was provided. This flask was charged with 340 g of n-heptane, and 0.92 g of a sucrose stearate having an HLB of 3 (produced by Mitsubishi-Kagaku Foods Corporation, Ryoto sugar ester S-370) and 0.92 g of a maleic anhydride-modified ethylene-propylene copolymer (produced by Mitsui Chemicals, Inc., Hi-wax 1105A) were added thereto. The temperature was raised to 80° C under stirring to dissolve the surfactant, and thereafter the solution was cooled to 50° C.

Separately, a 500-mL Erlenmeyer flask was charged with 92 g (1.02 mol) of 80% by mass acrylic acid aqueous solution, and 146.0 g of 21% by mass sodium hydroxide aqueous solution was added dropwise thereto under external cooling to neutralize 75 % by mol. Thereafter, 0.92 g of hydroxyethylcellulose (produced by Sumitomo Seika Chemicals Co., Ltd., HEC AW-15F) as a thickener, 0.11 g (0.41 mmol) of potassium persulfate as a radical polymerization initiator and 9.2 mg (0.06 mmol) of N,N'-methylenebisacrylamide as an internal crosslinking agent were added thereto to and dissolved, thereby preparing an aqueous monomer solution for the first step.

Setting the rotation speed of the stirrer at 600 r/min, the entire amount of aqueous monomer solution for the first step was added to the separable flask, and the inside of the system was sufficiently replaced with nitrogen. Thereafter, the flask was immersed in a water bath at 70° C to raise the temperature, and the first-step polymerization was carried out for 30 minutes, followed by cooling to room temperature, thereby obtaining polymerized liquid slurry for the first step. (Note that this polymerized slurry was subjected to azeotropic distillation of water and n-heptane using an oil bath at 120°C to distill off only water to the outside of the system. Subsequently, n-heptane was evaporated, and the slurry was dried. The resulting spherical primary particles had a median particle size of 160 $\mu$m.)

Separately, another 500-mL Erlenmeyer flask was charged with 128.8 g (1.43 mol) of 80% by mass acrylic acid aqueous solution, and 159.0 g of 27% by mass sodium hydroxide aqueous solution was added dropwise thereto under external cooling to neutralize 75 % by mol. Thereafter, 0.16 g (0.59 mmol) of potassium persulfate as a radical polymerization initiator and 12.9 mg (0.08 mmol) of N,N'-methylenebisacrylamide as an internal crosslinking agent were added thereto to and dissolved, thereby preparing an aqueous monomer solution for the second step.

After the rotation speed of the stirrer was changed to 1,000 r/min and the inside of the system was cooled to 21°C, the entire amount of aqueous monomer solution for the second step was added to the polymerized liquid slurry for the first step, and the inside of the system was sufficiently replaced with nitrogen. Thereafter, the flask was again immersed in a water bath at 70° C to raise the temperature, and the second-step polymerization was carried out for 30 minutes.

After the second-step polymerization, the temperature was raised using an oil bath at 125°C, and the slurry was subjected to azeotropic distillation of water and n-heptane to distill off 222 g of water to the outside of the system while refluxing n-heptane. Thereafter, 3.97 g (0.46 mmol) of a 2% by mass aqueous solution of ethylene glycol diglycidyl ether was added thereto, and the mixture was maintained at 80°C for 2 hours. Subsequently, n-heptane was evaporated, and the slurry was dried, thereby obtaining 231.3 g of water-absorbent resin (D) in the form of agglomeration of spherical particles. The water-absorbent resin had a median particle size of 434 $\mu$m and a water content of 7.9%. Table 1 shows the measurement results of each property.

Example 5

The same procedure as in Example 4 was repeated except that the rotation speed of the stirrer for the first step was changed to 500 r/min and that the amount of dehydration by refluxing n-heptane after the second-step polymerization was changed to 213 g, thereby obtaining 232.8 g of water-absorbent resin (E) in the form of agglomeration of spherical particles. The primary particles had a median particle size of 230 $\mu$m, and the water-absorbent resin had a median particle size of 458 $\mu$m and a water content of 10.0%. Table 1 shows the measurement results of each property.

Example 6

The same procedure as in Example 5 was repeated except that the rotation speed of the stirrer for the first step was changed to 400 r/min, thereby obtaining 234.2 g of water-absorbent resin (F) in the form of agglomeration of spherical particles. The primary particles had a median particle size of 250 $\mu$m, and the water-absorbent resin had a median particle size of 470 $\mu$m and a water content of 7.2%. Table 1 shows the measurement results of each property.

Comparative Example 1

The same procedure as in Example 1 was repeated except that the rotation speed of the stirrer for the first step was changed to 500 r/min and that the amount of dehydration by refluxing n-heptane after the second-step polymerization was changed to 230 g, thereby obtaining 229.6 g of water-absorbent resin (G) in the form of agglomeration of spherical particles. The primary particles had a median particle size of 90 $\mu$m, and the water-absorbent resin had a median particle size of 364 $\mu$m and a water content of 7.0%. Table 1 shows the measurement results of each property.

Comparative Example 2

The same procedure as in Example 4 was repeated except that the rotation speed of the stirrer for the first step was changed to 350 r/min and that the amount of dehydration by refluxing n-heptane after the second-step polymerization was changed to 213 g, thereby obtaining 227.6 g of water-absorbent resin (H) in the form of agglomeration of spherical particles. The primary particles had a median particle size of 280 $\mu$m, and the water-absorbent resin had a median particle size of 519 $\mu$m and a water content of 6.9%. Table 1 shows the measurement results of each property.

Comparative Example 3

The same procedure as in Example 2 was repeated except that the amount of dehydration by refluxing n-heptane after the second-step polymerization was changed to 230 g, thereby obtaining 231.7 g of water-absorbent resin (I) in the form of agglomeration of spherical particles. The primary particles had a median particle size of 130 $\mu$m, and the water-absorbent resin had a median particle size of 420 $\mu$m and a water content of 6.4%. Table 1 shows the measurement results of each property.

Comparative Example 4

The same procedure as in Example 5 was repeated except that the amount of dehydration by refluxing n-heptane after the second-step polymerization was changed to 233 g, thereby obtaining 233.4 g of water-absorbent resin (J) in the form of agglomeration of spherical particles. The primary particles had a median particle size of 230 μm, and the water-absorbent resin had a median particle size of 448 μm and a water content of 5.4%. Table 1 shows the measurement results of each property.

[Table 1]

| | Median particle size of primary particles (μm) | Water-retention capacity of saline solution (g/g) | Water-absorption rate of saline solution (second) | flow-through rate of 0.69% by mass sodium chloride aqueous solution (g/10min) |
|---|---|---|---|---|
| Example 1 | 110 | 27.7 | 48 | 227 |
| Example 2 | 130 | 28.2 | 81 | 286 |
| Example 3 | 130 | 25.8 | 77 | 392 |
| Example 4 | 160 | 28.8 | 85 | 376 |
| Example 5 | 230 | 28.5 | 127 | 616 |
| Example 6 | 250 | 28.3 | 159 | 709 |
| Comparative Example 1 | 90 | 25.8 | 35 | 182 |
| Comparative Example 2 | 280 | 28.6 | 200 | 811 |
| Comparative Example 3 | 130 | 31.6 | 44 | 82 |
| Comparative Example 4 | 230 | 32.4 | 154 | 100 |

As is clear from Table 1, the water-absorbent resins in Examples 1 to 6 have appropriate water-retention capacity of saline solution and water-absorption rate of saline solution, and provide an excellent flow-through rate of 0.69% by mass sodium chloride aqueous solution.

On the other hand, of the Comparative Examples, the one (Comparative Example 1) whose primary particles have a small median particle size was as follows: the flow-through rate of 0.69% by mass sodium chloride aqueous solution was slow and the water-absorption rate of saline solution was fast. The one (Comparative Example 2) whose primary particles have a large median particle size was as follows: the flow-through rate of 0.69% by mass sodium chloride aqueous solution was fast but the water-absorption rate of saline solution was slow. The ones (Comparative Examples 3 and 4) whose primary particles have a moderate median particle size and a high physiological saline absorption rate water-retention capacity of saline solution were as follows: the flow-through rate of 0.69% by mass sodium chloride aqueous solution was fast.

Next, absorbent materials and absorbent articles were prepared using the water-absorbent resins obtained in Examples 1 to 6 and Comparative Examples 1 to 4.

Example 7

10 g of the water-absorbent resin (A) obtained in Example 1 and 10 g of crushed pulp were dry-blended using a mixer. The resulting mixture was sprayed on tissue paper (1 g) 40 cm × 12 cm in size. Subsequently, another tissue paper having the same size and weight was placed over the tissue paper in an overlapped manner to form a sheet, and the entire sheet was pressed under a load of 196 kPa for 30 seconds, thereby obtaining an absorbent material. The resulting absorbent material was sandwiched between a polyethylene air-through porous liquid-permeable sheet 40 cm × 12 cm in size and 20 g/m$^2$ in basis weight, and a polyethylene non-permeable sheet having the same size and weight, thereby obtaining an absorbent article that uses the absorbent material.

Example 8

The procedure of Example 7 was repeated except that the water-absorbent resin (B) obtained in Example 2 was used, thereby obtaining an absorbent material and an absorbent article that uses the same.

14

Example 9

The procedure of Example 7 was repeated except that the water-absorbent resin (C) obtained in Example 3 was used, thereby obtaining an absorbent material and an absorbent article that uses the same.

Example 10

The procedure of Example 7 was repeated except that the water-absorbent resin (D) obtained in Example 4 was used, thereby obtaining an absorbent material and an absorbent article that uses the same.

Example 11

The procedure of Example 7 was repeated except that the water-absorbent resin (E) obtained in Example 5 was used, thereby obtaining an absorbent material and an absorbent article that uses the same.

Example 12

The procedure of Example 7 was repeated except that the water-absorbent resin (F) obtained in Example 6 was used, thereby obtaining an absorbent material and an absorbent article that uses the same.

Comparative Example 5

The procedure of Example 7 was repeated except that the water-absorbent resin (G) obtained in Comparative Example 1 was used, thereby obtaining an absorbent material and an absorbent article that uses the same.

Comparative Example 6

The procedure of Example 7 was repeated except that the water-absorbent resin (H) obtained in Comparative Example 2 was used, thereby obtaining an absorbent material and an absorbent article that uses the same.

Comparative Example 7

The procedure of Example 7 was repeated except that the water-absorbent resin (I) obtained in Comparative Example 3 was used, thereby obtaining an absorbent material and an absorbent article that uses the same.

Comparative Example 8

The procedure of Example 7 was repeated except that the water-absorbent resin (J) obtained in Comparative Example 4 was used, thereby obtaining an absorbent material and an absorbent article that uses the same.

The absorbent articles obtained above were evaluated following the methods described below. Table 2 shows the results.

<Evaluation of Absorbent Article>

(a) Preparation of Artificial Urine

Sodium chloride (60 g), calcium chloride dihydrate (1.8 g), magnesium chloride hexahydrate (3.6 g), and distilled water (q.s.) were placed in a 10-L container, and completely dissolved. Next, 15 g of 1% by mass poly(oxyethylene)isooctylphenyleter aqueous solution was added to the solution, and distilled water was further added thereto to adjust the total mass of the aqueous solution to 6,000 g. Subsequently, the solution was colored with a small amount of Blue No. 1, thereby preparing artificial urine.

(b) Liquid Permeation time

A cylindrical cylinder having an inside diameter of 3 cm was placed near the center of the absorbent article, and 50 mL of artificial urine was poured into the cylinder at once. At the same time, using a stopwatch, the time taken for the artificial urine to completely disappear from the cylinder was measured as a first liquid permeation time(seconds). Next, the cylinder was removed, and the absorbent article was stored as is. At 30 minutes and 60 minutes after the start of the first pouring of the artificial urine, the cylinder was placed at the same position as the first time, and the same procedure was carried out to measure the liquid permeation time (seconds) for the second and third times. The total time in seconds of the first to third procedures was regarded as the total liquid permeation time.

(c) Amount of Re-wet

After the elapse of 60 minutes from the completion of the measurement of the liquid permeation time, about 80 sheets of 10 cm$^2$ filter paper whose mass had been measured in advance ((We (g), about 70 g) were placed near the position for pouring the artificial urine on the absorbent article, and a 5-kg weight having a bottom area of 10 cm $\times$ 10 cm was placed on the filter paper. After the load was applied for 5 minutes, the mass of filter paper (Wf (g)) was measured, and the increased mass was regarded as the amount of re-wet(g).

$$\texttt{Amount of re-wet of the liquid (g) = Wf-We}$$

(d) Expansion Length

Within 5 minutes after the completion of the measurement of the amount of re-wet, the dimension of the longitudinal expansion (cm) of each absorbent article into which the artificial urine was permeated was measured. Note that the decimal point was rounded off to the nearest whole number.

[Table 2]

| | Resin Used | Liquid Permeation Time (Seconds) | | | | Amount of Re-wet (g) | Expansion Length (cm) |
|---|---|---|---|---|---|---|---|
| | | 1 | 2 | 3 | total | | |
| Example 7 | A | 20 | 17 | 22 | 59 | 26.2 | 19 |
| Example 8 | B | 21 | 17 | 19 | 27 | 25.9 | 20 |
| Example 9 | C | 20 | 16 | 17 | 53 | 25.4 | 21 |
| Example 10 | D | 21 | 16 | 18 | 55 | 26 | 20 |
| Example 11 | E | 21 | 14 | 16 | 52 | 27.1 | 22 |
| Example 12 | F | 21 | 13 | 15 | 50 | 29.8 | 24 |
| Comparative Example 5 | G | 21 | 17 | 27 | 65 | 26.5 | 18 |
| Comparative Example 6 | H | 21 | 12 | 16 | 50 | 35.9 | 24 |
| Comparative Example 7 | I | 21 | 18 | 29 | 69 | 29.3 | 17 |
| Comparative Example 8 | J | 20 | 17 | 28 | 66 | 32.4 | 17 |

As is clear from Table 2, the absorbent articles of Examples 7 to 12 that respectively use the water-absorbent resins (A) to (F), which are excellent in the water-retention capacity of saline solution, water-absorption rate of saline solution, and flow-through rate of 0.69% by mass sodium chloride aqueous solution, have a short liquid permeation time and a small re-wet.
On the other hand, of the Comparative Examples, the ones (Comparative Examples 5 and 6) that have poor properties in terms of water-absorption rate of saline solution and flow-through rate of 0.69% by mass sodium chloride aqueous solution, and the ones (Comparative Examples 7 and 8) that are high in the water-retention capacity of saline solution and poor in the property of flow-through rate of 0.69% by mass sodium chloride aqueous solution due to unsuitable median particle size of the primary particles, resulted in absorbent articles having poor properties in terms of liquid permeation time and amount of re-wet.

Industrial Applicability

The water-absorbent resin of the present invention provides an excellent flow-through property, and can be suitably used for absorbent materials and absorbent articles that use the same, in particular, for hygienic materials.

Reference Symbol List

11  Tank
12  Glass tube for static pressure adjustment
13  0.69% by mass sodium chloride aqueous solution
14  L-shaped tube having a cock
15  Cock
20  Cylindrical container
21  Piston-type weight
22  Cylinder
23  Hole
24  Glass filter
25  Swollen gel
26  Stainless metal guaze
31  Funnel
32  Support board
33  Collecting Container
34  Even balance

**Claims**

1.  A method of producing a water-absorbent resin, comprising the steps of:

    (1) polymerizing at least one water-soluble ethylenically unsaturated monomer in the presence of a dispersion stabilizer according to a reversed-phase suspension polymerization method to form a slurry in which primary particles are dispersed, the primary particles having a median particle size of 100 to 250 μm, and
    (2-1) cooling the slurry obtained in Step (1) to precipitate a portion of the dispersion stabilizer contained in the slurry,
    (2-2) adding at least one water-soluble ethylenically unsaturated monomer to the slurry obtained in Step (2-1) to perform a polymerization reaction (reversed-phase suspension polymerization method), thereby agglomerating the primary particles dispersed in the slurry, to produce a water-absorbent resin,

    wherein the water-absorbent resin has a water-retention capacity of saline solution of 30 g/g or less, wherein the saline solution is a 0.9% by mass sodium chloride aqueous solution, and the water-absorbent resin has a flow-through rate of 0.69% by mass sodium chloride aqueous solution of 200 g/10 minutes or more.

2.  The method of producing a water-absorbent resin according to claim 1, wherein the water-absorbent resin has a water-absorption rate of saline solution of 45 to 180 seconds.

3.  The method of producing a water-absorbent resin according to claim 1 or 2, wherein a dispersion stabilizer is at least one member selected from the group consisting of sucrose fatty acid esters and polyglycerol fatty acid esters.

4.  The method of producing a water-absorbent resin according to any one of claims 1 to 3, wherein the water-soluble ethylenically unsaturated monomer is at least one member selected from the group consisting of (meth)acrylic acid and a salt thereof.

5.  The method of producing a water-absorbent resin according to any one of claims 1 to 4, wherein the water-soluble ethylenically unsaturated monomer used in step (2-2) is added in an amount of 50 to 300 parts by mass, relative to 100 parts by mass of the water-soluble ethylenically unsaturated monomer used in step (1).

6. The method of producing a water-absorbent resin according to any one of claims 1 to 5, wherein the water-absorbent resin has a median particle size of 250 μm or more and 600 μm or less.

7. The method of producing a water-absorbent resin according to any one of claims 1 to 6, further comprising the step of (3) adding a post-crosslinking agent after the primary particles are agglomerated, wherein the post-crosslinking agent is added in the presence of 1 to 400 parts by mass of water, relative to 100 parts by mass of the total amount of the water-soluble ethylenically unsaturated monomer.

8. A water-absorbent resin obtainable by the method of any one of claims 1 to 7.

9. An absorbent material that comprises a hydrophilic fiber and the water-absorbent resin according to claim 8.

10. An absorbent article including the absorbent material according to claim 9 between a liquid-permeable sheet and a liquid-impermeable sheet.


**Patentansprüche**

1. Ein Verfahren zur Herstellung eines wasserabsorbierenden Harzes, umfassend die Schritte:

   (1) Polymerisieren mindestens eines wasserlöslichen ethylenisch ungesättigten Monomers in Gegenwart eines Dispersionsstabilisators, gemäß eines Verfahrens zur Umkehrphasen-Suspensionspolymerisation, um eine Aufschlämmung zu bilden, in der Primärteilchen dispergiert sind, wobei die Primärteilchen eine mittlere Teilchengröße von 100 bis 250 μm aufweisen, und
   (2-1) Kühlen der in Schritt (1) erhaltenen Aufschlämmung, um einen Teil des in der Aufschlämmung enthaltenen Dispersionsstabilisators zu fällen,
   (2-2) Zugeben mindestens eines wasserlöslichen ethylenisch ungesättigten Monomers zu der in Schritt (2-1) erhaltenen Aufschlämmung, um eine Polymerisationsreaktion durchzuführen (Verfahren zur Umkehrphasen-Suspensionspolymerisation), wodurch die in der Aufschlämmung dispergierten Primärteilchen agglomeriert werden, um ein wasserabsorbierendes Harz herzustellen,

   wobei das wasserabsorbierende Harz ein Wasserrückhaltevermögen einer Kochsalzlösung von 30 g/g oder weniger aufweist, wobei die Kochsalzlösung eine wässrige Natriumchloridlösung mit 0,9 Massen-% ist und das wasserabsorbierende Harz eine Durchflussgeschwindigkeit von 200 g/10 Minuten oder mehr, bei einer wässrigen Natriumchloridlösung mit 0,69 Massen-%, aufweist.

2. Das Verfahren zur Herstellung eines wasserabsorbierenden Harzes gemäß Anspruch 1, wobei das wasserabsorbierende Harz eine Wasserabsorptionsgeschwindigkeit der Kochsalzlösung von 45 bis 180 Sekunden aufweist.

3. Das Verfahren zur Herstellung eines wasserabsorbierenden Harzes gemäß Anspruch 1 oder 2, wobei ein Dispersionsstabilisator mindestens ein Element, ausgewählt aus der Gruppe bestehend aus Saccharosefettsäureestern und Polyglycerinfettsäureestern, ist.

4. Das Verfahren zur Herstellung eines wasserabsorbierenden Harzes gemäß einem der Ansprüche 1 bis 3, wobei das wasserlösliche ethylenisch ungesättigte Monomer mindestens ein Element, ausgewählt aus der Gruppe bestehend aus (Meth)acrylsäure und einem Salz davon, ist.

5. Das Verfahren zur Herstellung eines wasserabsorbierenden Harzes gemäß einem der Ansprüche 1 bis 4, wobei das in Schritt (2-2) verwendete wasserlösliche ethylenisch ungesättigte Monomer in einer Menge von 50 bis 300 Masseteilen, bezogen auf 100 Masseteile des in Schritt (1) verwendeten wasserlöslichen ethylenisch ungesättigten Monomers, zugegeben wird.

6. Das Verfahren zur Herstellung eines wasserabsorbierenden Harzes gemäß einem der Ansprüche 1 bis 5, wobei das wasserabsorbierende Harz eine mittlere Teilchengröße von 250 μm oder mehr und 600 μm oder weniger aufweist.

7. Das Verfahren zur Herstellung eines wasserabsorbierenden Harzes gemäß einem der Ansprüche 1 bis 6, ferner umfassend den Schritt:

(3) Zugeben eines Nachvernetzungsmittels, nachdem die Primärteilchen agglomeriert sind, wobei das Nachvernetzungsmittel in Gegenwart von 1 bis 400 Masseteilen Wasser, bezogen auf 100 Masseteile der Gesamtmenge des wasserlöslichen ethylenisch ungesättigten Monomers, zugegeben wird.

8. Ein wasserabsorbierendes Harz, das durch das Verfahren gemäß einem der Ansprüche 1 bis 7 erhältlich ist.

9. Ein absorbierendes Material, das eine hydrophile Faser und das wasserabsorbierende Harz gemäß Anspruch 8 umfasst.

10. Ein absorbierender Gegenstand, der das absorbierende Material gemäß Anspruch 9 zwischen einer flüssigkeitsdurchlässigen Lage und einer flüssigkeitsundurchlässigen Lage beinhaltet.

**Revendications**

1. Procédé de production d'une résine absorbant l'eau, comprenant les étapes de :

(1) polymérisation d'au moins un monomère éthyléniquement insaturé soluble dans l'eau en présence d'un stabilisant de dispersion selon un procédé de polymérisation de suspension à phase inversée pour former une boue dans laquelle sont dispersées des particules primaires, les particules primaires ayant une taille de particule moyenne de 100 à 250 $\mu$m, et
(2-1) refroidissement de la boue de l'étape (1) pour précipiter une partie du stabilisant de dispersion contenu dans la boue,
(2-2) ajout d'au moins un monomère éthyléniquement insaturé soluble dans l'eau à la boue obtenue à l'étape (2-1) pour réaliser une réaction de polymérisation (procédé de polymérisation de suspension à phase inversée), regroupant ainsi les particules primaires dispersées dans la boue, pour produire une résine absorbant l'eau,

dans lequel la résine absorbant l'eau a une capacité de rétention d'eau de solution saline de 30 g/g ou moins, dans lequel la solution saline est une solution aqueuse à 0,9 % en masse de chlorure de sodium, et la résine absorbant l'eau a un débit d'écoulement de solution aqueuse à 0,69 % en masse de chlorure de sodium de 200 g/10 minutes ou plus.

2. Procédé de production d'une résine absorbant l'eau selon la revendication 1, dans lequel la résine absorbant l'eau a une vitesse d'absorption d'eau de solution saline de 45 à 180 secondes.

3. Procédé de production d'une résine absorbant l'eau selon la revendication 1 ou 2, dans lequel un stabilisant de dispersion est au moins un élément sélectionné dans le groupe constitué d'esters d'acides gras de saccharose et d'esters d'acides gras de polyglycérol.

4. Procédé de production d'une résine absorbant l'eau selon l'une quelconque des revendications 1 à 3, dans lequel le monomère éthyléniquement insaturé soluble dans l'eau est au moins un élément sélectionné dans le groupe constitué d'acide (méth)acrylique et d'un sel de celui-ci.

5. Procédé de production d'une résine absorbant l'eau selon l'une quelconque des revendications 1 à 4, dans lequel le monomère éthyléniquement insaturé soluble dans l'eau utilisé à l'étape (2-2) est ajouté en une quantité de 50 à 300 parts en masse, par rapport à 100 parts en masse du monomère éthyléniquement insaturé soluble dans l'eau utilisé à l'étape (1).

6. Procédé de production d'une résine absorbant l'eau selon l'une quelconque des revendications 1 à 5, dans lequel la résine absorbant l'eau a une taille de particule moyenne de 250 $\mu$m ou plus et de 600 $\mu$m ou moins.

7. Procédé de production d'une résine absorbant l'eau selon l'une quelconque des revendications 1 à 6, comprenant en outre l'étape de
(3) ajout d'un agent de post-réticulation après le regroupement des particules primaires, dans lequel l'agent de post-réticulation est ajouté en présence de 1 à 400 parts en masse d'eau, par rapport à 100 parts en masse de la quantité totale du monomère éthyléniquement insaturé soluble dans l'eau.

8. Résine absorbant l'eau susceptible d'être obtenue par le procédé selon l'une quelconque des revendications 1 à 7.

9. Matériau absorbant qui comprend une fibre hydrophile et la résine absorbant l'eau selon la revendication 8.

10. Article absorbant incluant le matériau absorbant selon la revendication 9 entre une feuille perméable aux liquides et une feuille imperméable aux liquides.

Figure 1

**EP 2 607 383 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H3227301 B
- JP H5017509 W
- JP H9012613 B
- JP H9077810 W
- EP 2184300 A1